# EUROPEAN PATENT APPLICATION

(11) **EP 2 842 947 A1**
(43) Date of publication of application: **04.03.2015**
(21) Application number: 13777470.9
(22) Date of filing: 05.03.2013
(51) Int. Cl.: C07D 249/08

(54) **METHOD FOR PRODUCING TRIAZOLYL METHYL CYCLOALKANOL DERIVATIVE AND TRIAZOLYL METHYL CYCLOALKANOL DERIVATIVE-CONTAINING COMPOSITION**

(30) Priority: 18.04.2012 JP 2012095015
(71) Applicant: Kureha Corporation, Chuo-ku Tokyo 103-8552 (JP)
(72) Inventor: OOHASHI, Takashi, Tokyo 103-8552 (JP); ARAKI, Nobuyuki, Tokyo 103-8552 (JP); SHIMOKAWARA, Takashi, Tokyo 103-8552 (JP)
(74) Representative: Hoffmann Eitle
(86) International application number: PCT/JP2013/055994
(87) International publication number: WO 2013/157311

(57) **Abstract**

Generation of by-product is reduced by producing a triazolylmethyl cycloalkanol derivative by reacting a cycloalkanone derivative and an alkali metal salt of 1,2,4-triazole in the presence of a sulfur ylide at a reaction temperature of higher than 110°C and 140°C or lower.

## Description

### TECHNICAL FIELD

The present invention relates to a method for producing a triazolylmethyl cycloalkanol derivative and particularly relates to a method for producing a triazolylmethyl cycloalkanol derivative from a cycloalkanone derivative.

### BACKGROUND ART

Certain types of 2-substituted-5-benzyl-1-azolylmethyl cyclopentanol derivatives have been known to exhibit bactericidal activity. As a method for producing a 2-substituted-5-benzyl-1-azolylmethyl cyclopentanol derivative, Patent Document 1 discloses a method for azolylmethylating a cycloalkanone derivative using an alkali metal salt of 1,2,4-triazole in the presence of ylide.

### CITATION LIST

### Patent Literature

Patent Document 1: Japanese Unexamined Patent Application Publication No. H01-301664A (published on December 5, 1989)

### SUMMARY OF INVENTION

### Technical Problem

As a result of research conducted by the present inventors, it was found that, in addition to a 1,2,4-triazole form of an azolylmethyl cycloalkanol derivative which is the target compound, a 1,3,4-triazole form is also produced as a by-product in conventional methods for producing azolylmethyl cycloalkanol derivative. Thus, reducing the production of by-products so as to increase the purity of the target compound has been desired.

Therefore, the present invention has been completed in light of the foregoing problem, and an object of the present invention is to provide a method for producing an azolylmethyl cycloalkanol derivative by which production of a by-product is reduced.

### Solution To Problem

As a result of diligent research on various methods, the present inventors discovered that the production of by-products can be suppressed by performing a reaction at a higher temperature than that of conventional methods, and thus completed the invention of the present application. That is, in order to solve the above problems, the method for producing a triazolylmethyl cycloalkanol derivative according to the present invention is a method for producing a triazolylmethyl cycloalkanol derivative, in which a cycloalkanone derivative represented by general formula (I):

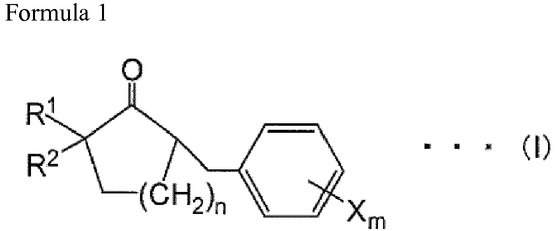

(in formula (I), R¹ represents a hydrogen atom or an alkyl group or haloalkyl group having from 1 to 5 carbons, R² represents a hydrogen atom or an alkyl group having 1 or 2 carbons, X represents a halogen atom, an alkyl group having from 1 to 4 carbons, a phenyl group, -CN, -CF₃, or -NO₂, m represents an integer of 1 to 5, and n represents an integer of 1 to 3, and if m is 2 or greater, the plurality of X may be different each other) is azolylmethylated to produce a triazolylmethyl cycloalkanol derivative represented by general formula (II):

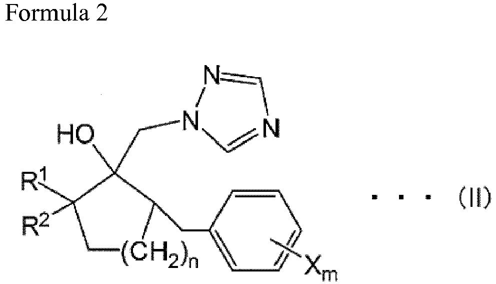

(in formula (II), R¹, R², X, m, and n are the same as R¹, R², X, m, and n in formula (I) respectively);
the method for producing a triazolylmethyl cycloalkanol derivative comprising a step of reacting, in a solvent, the cycloalkanone derivative with an alkali metal salt of 1,2,4-triazole in the presence of sulfur ylide; a reaction temperature in the reacting step being higher than 110°C and 140°C or lower.

### Effect of the Invention

According to the method for producing a triazolylmethyl cycloalkanol derivative of the present invention, 1,2,4-triazole form of triazolylmethyl cycloalkanol derivative, which is the target to be produced, can be produced while the production of 1,3,4-triazole form of the triazolylmethyl cycloalkanol derivative, which is a byproduct, is suppressed.

### DETAILED DESCRIPTION

An embodiment of the method for producing a triazolylmethyl cycloalkanol derivative according to the present invention will be described hereinafter.

The present invention is a method for producing a triazolylmethyl cycloalkanol derivative, in which a cycloalkanone derivative represented by general formula (I):

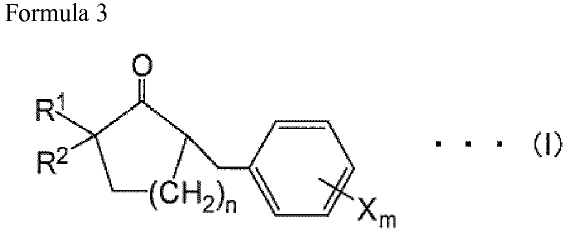

(in formula (I), R¹ represents a hydrogen atom or an alkyl group or haloalkyl group having from 1 to 5 carbons, R² represents a hydrogen atom or an alkyl group having 1 or 2 carbons, X represents a halogen atom, an alkyl group having from 1 to 4 carbons, a phenyl group, -CN, -CF₃, or -NO₂, m represents an integer of 1 to 5, and n represents an integer of 1 to 3, and if m is 2 or greater, the plurality of X may be different each other)
is azolylmethylated to produce a triazolylmethyl cycloalkanol derivative represented by general formula (II):

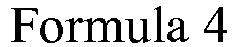

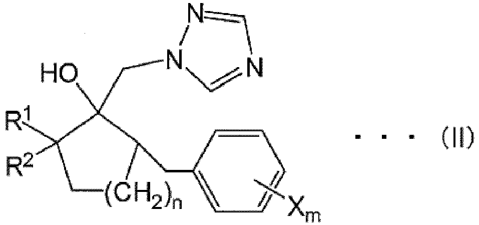

(in formula (II), R¹, R², X, m, and n are the same as R¹, R², X, m, and n in formula (I) respectively).

R¹ represents a hydrogen atom or an alkyl group or haloalkyl group having from 1 to 5 carbons. Specific examples of the alkyl group having from 1 to 5 carbons include a methyl group, ethyl group, n-propyl group, isopropyl group, n-butyl group, isobutyl group, sec-butyl group, tert-butyl group, pentyl group, 1-methylbutyl group, 2-methylbutyl group, 1-ethylpropyl group, and the like. Of these, a methyl group, ethyl group, and isopropyl group are preferable, and a methyl group is more preferable. Examples of the haloalkyl group having from 1 to 5 carbons include above-described alkyl groups in which at least one hydrogen atom has been replaced with halogen atom(s). Examples of the halogen atom are preferably a fluorine atom, chlorine atom, and bromine atom.

R² represents a hydrogen atom or an alkyl group having 1 or 2 carbons, and specifically represents a hydrogen atom, a methyl group, or an ethyl group. Of these, R² is preferably a hydrogen atom or a methyl group, and a methyl group is more preferable.

X represents a halogen atom, an alkyl group having from 1 to 4 carbons, a phenyl group, -CN, -CF₃, or -NO₂. Examples of the halogen atom include a fluorine atom, chlorine atom, bromine atom, and the like. Specific examples of the alkyl group having from 1 to 4 carbons include a methyl group, ethyl group, n-propyl group, isopropyl group, n-butyl group, isobutyl group, sec-butyl group, tert-butyl group, and the like. Of these, X is preferably a chlorine atom or a bromine atom, and a chlorine atom is more preferable.

m represents an integer of 1 to 5, and, if m is 2 or greater, the plurality of X may be the same or different each other. m is preferably 1 or 2 and more preferably 1. When m is an integer of 1 to 4, X may be positioned at any of the positions 2 to 6 of a benzene ring. When m is 1, a position forming 4-substituted benzyl is preferable.

n represents an integer of 1 to 3, and n is preferably 1. That is, the cycloalkanone derivative represented by general formula (I) is a cyclopentanone derivative, cyclohexanone derivative, or cycloheptanone derivative, and preferably a cyclopentanone derivative.

In the method for producing a triazolylmethyl cycloalkanol derivative of the present embodiment, the cycloalkanone derivative (hereinafter called "cycloalkanone derivative (I)") represented by general formula (I) is reacted, in a solvent, with an alkali metal salt of 1,2,4-triazole in the presence of sulfur ylide at a reaction temperature of higher than 110°C and 140°C or lower.

A solvent selected from (i) to (iii) below is used as the solvent: (i) a polar solvent having an amide bond, (ii) dimethyl sulfoxide, and (iii) a mixed solvent of a polar solvent having an amide bond or dimethyl sulfoxide and alcohol having from 1 to 5 carbons.

Examples of the polar solvent having an amide bond include N-methyl-2-pyrrolidone, dimethylformamide, and dimethylacetamide. Examples of the alcohol having from 1 to 5 carbons include a methanol, ethanol, n-propanol, isopropanol, n-butanol, tert-butanol, and the like, and a tert-butanol is preferable. The solvent is preferably N-methyl-2-pyrrolidone or a mixed solvent of N-methyl-2-pyrrolidone and tert-butanol.

Examples of the sulfur ylide include sulfonium ylide and sulfoxonium ylide. Examples of the sulfonium ylide include dimethyl sulfonium methylide. In addition, examples of the sulfoxonium ylide include dimethyl sulfoxonium methylide.

The used amount of the sulfur ylide is, for example, from 1.0 to 5.0 times, and preferably from 1.0 to 2.0 times, the molar quantity of the cycloalkanone derivative (I).

Examples of the alkali metal salt of 1,2,4-triazole include sodium 1,2,4-triazole salt, potassium 1,2,4-triazole salt, lithium 1,2,4-triazole salt, and the like.

The used amount of the alkali metal salt of 1,2,4-triazole is, for example, from 1.0 to 5.0 times, and preferably from 1.0 to 2.0 times, the molar quantity of the cycloalkanone derivative (I).

The reaction time is, for example, from 3 to 20 hours, and in many cases, from 5 to 10 hours. In addition, the reaction is preferably performed under stirring.

The reaction temperature is a temperature of higher than 110°C and 140°C or lower. Of these, the reaction temperature is preferably a temperature of higher than 120°C or a temperature of 130°C or lower. The reaction temperature is more preferably a temperature of higher than 120°C and 130°C or lower.

In the reaction in the step described above, in addition to the cycloalkanol derivative represented in general formula (II) (1,2,4-triazole form; hereinafter "cycloalkanol derivative (II)") described above which is the target compound, cycloalkanol derivative represented by general formula (III) (1,3,4-triazole form; hereinafter "cycloalkanol derivative (III)") described below is produced.

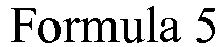

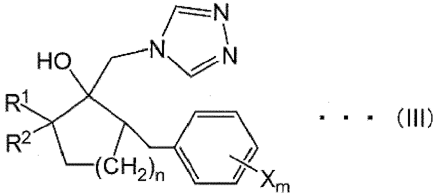

In formula (III), R¹, R², X, m, and n are the same as R¹, R², X, m, and n in formula (I) respectively. In order to increase the content of the cycloalkanol derivative (II) in the reaction mixture, produced amount of the cycloalkanol derivative (III) needs to be reduced.

Here, by setting the reaction temperature to be a temperature higher than 110°C, the produced amount of the cycloalkanol derivative (III) can be suppressed to low. On the other hand, in order to set the reaction temperature to 140°C or greater, it is required to prepare expensive reaction equipment since a heat medium other than steam is required and the number of supplemental equipment is increased. Therefore, the reaction temperature is preferably 140°C or lower.

A first aspect of the production method in the present embodiment is a method to perform a reaction by combining a solution in which the cycloalkanone derivative (I) is dissolved in the solvent described above with a solution in which a separately prepared sulfur ylide and an alkali metal salt of 1,2,4-triazole are added in the solvent described above. The sulfur ylide may be added at once or may be added dividedly in 3 to 15 times. Alternatively, the sulfur ylide may be added dropwise over 3 to 7 hours.

Note that the sulfur ylide may be a sulfur ylide that is produced within the reaction system. Specifically, the sulfur ylide may be sulfonium ylide or sulfoxonium ylide generated in the reaction system by subjecting sulfonium halide or sulfoxonium halide to a reaction with a base in the reaction system. Therefore, a second aspect of the present embodiment is a method including adding the cycloalkanone derivative (I) and the alkali metal salt of 1,2,4-triazole to the solvent, intermittently adding sulfonium halide or sulfoxonium halide and the base in the mixture, and reacting the mixture at a reaction temperature of higher than 110°C and 140°C or lower. In addition, a third aspect of the present embodiment is a method including adding the cycloalkanone derivative (I), the alkali metal salt of 1,2,4-triazole, and the base to the solvent, intermittently adding sulfonium halide or sulfoxonium halide in the mixture, and reacting the mixture at a reaction temperature of higher than 110°C and 140°C or lower.

Examples of the sulfonium halide include trimethylsulfonium halide. Specific examples thereof include trimethylsulfonium iodide, trimethylsulfonium bromide, trimethylsulfonium chloride, and the like. In addition, examples of the sulfoxonium halide include trimethylsulfoxonium halide. Specific examples thereof include trimethylsulfoxonium iodide, trimethylsulfoxonium bromide, trimethylsulfoxonium chloride, and the like.

Examples of the base include carbonates of alkali metal such as sodium carbonate and potassium carbonate; hydroxides of alkali metal such as sodium hydroxide and potassium hydroxide; alkoxides of alkali metal such as sodium methoxide, sodium ethoxide, sodium tert-butoxide, and potassium tert-butoxide; alkali metal hydrides such as sodium hydride and potassium hydride; alkyl compounds of alkali metal such as n-butyllithium; and triethylamine, pyridine, and the like.

The total used amount of the sulfonium halide or sulfoxonium halide is, for example, from 1.0 to 5.0 times, and preferably from 1.0 to 2.0 times, the molar quantity of the cycloalkanone derivative (I).

The total used amount of the base is, for example, from 0.5 to 5.0 times, and preferably from 0.5 to 2.0 times, the molar quantity of the cycloalkanone derivative (I).

The number of the intermittent addition of sulfonium halide or sulfoxonium halide and a base in the second aspect, and the number of intermittent addition of sulfonium halide or sulfoxonium halide in the third aspect are not particularly limited as long as the number is sufficient to achieve a predetermined purpose. For example, each of the numbers may be from 2 to 20 times and preferably from 3 to 15 times.

In all of the aspects, the alkali metal salt of 1,2,4-triazole may be produced in the reaction system. The alkali metal salt of 1,2,4-triazole can be produced from 1,2,4-triazole and a base containing an alkali metal atom. Examples of the base containing an alkali metal atom include, among the bases described above, bases containing an alkali metal atom. In the case where an alkali metal salt of 1,2,4-triazole is produced within the same reaction system in the second and third aspects in which sulfur ylide is produced by reacting sulfonium halide or sulfoxonium halide and a base in the reaction system, the base containing an alkali metal atom may be the same as or different from the base used to produce the sulfur ylide. In the case where the same base is used, time and effort required to add the base can be reduced. In the case where a different base is used, a suitable base can be selected and used for each reaction. In the case where the alkali metal salt of 1,2,4-triazole is produced in the reaction system, a reaction equipment (storing equipment) for producing the alkali metal salt of 1,2,4-triazole in advance is not needed, thereby cost for the production can be reduced. Furthermore, since the alkali metal salt of 1,2,4-triazole has high moisture absorbency, it is preferable to produce the alkali metal salt of 1,2,4-triazole in the reaction system from the perspective of ease of handling.

Note that, in the second aspect, the cycloalkanol derivative (II) can be obtained in high yield.

In all of the aspects, the mixture obtained by the reaction is cooled after completion of the reaction, then added to ice-cold water, and extracted with an organic solvent, such as ethyl acetate, chloroform, methylene chloride, benzene, and toluene, to separate the organic phase. Thereafter, the organic phase is washed and dried. After drying, the solvent was removed by distillation under reduced pressure and the obtained residue was subjected to purification.

The reaction product and the purified product obtained after purification is a composition containing, in addition to the cycloalkanol derivative (II), a slight amount of cycloalkanol derivative (III). According to the method for producing a triazolylmethyl cycloalkanol derivative of the present embodiment, the content of the cycloalkanol derivative (III) in the composition is suppressed to a lower content compared to that of the product produced by conventional production method. Therefore, the triazolylmethyl cycloalkanol derivative-containing composition obtained by the method for producing a triazolylmethyl cycloalkanol derivative of the present invention is also in the scope of the present invention. In the triazolylmethyl cycloalkanol derivative-containing composition of the present invention, the content of the cycloalkanol derivative (III) is 5.0 wt.% or less of the amount of the cycloalkanol derivative (II). Note that the content of the cycloalkanol derivative (III) can be measured by, for example, gas chromatography.

As described above, according to the production method of the present embodiment, production of the cycloalkanol derivative (II) by which the production of the by-product is reduced can be achieved by performing the reaction at a higher reaction temperature than that of conventional methods. Therefore, the production of the by-product can be reduced in the production of the cycloalkanol derivative (II) without introducing a new step and without increasing the production cost.

As described above, the method for producing a triazolylmethyl cycloalkanol derivative according to the present invention is a method, in which a cycloalkanone derivative (I) is azolylmethylated to produce a cycloalkanol derivative (II). The method for producing a triazolylmethyl cycloalkanol derivative comprises a step of reacting, in a solvent, the cycloalkanone derivative (II) with an alkali metal salt of 1,2,4-triazole in the presence of sulfur ylide, and a reaction temperature in the reacting step is higher than 110°C and 140°C or lower.

In the method for producing a triazolylmethyl cycloalkanol derivative according to the present invention, the sulfur ylide in the reacting step is preferably sulfur ylide produced in the reaction system in the reacting step.

In the method for producing a triazolylmethyl cycloalkanol derivative according to the present invention, the sulfur ylide is preferably sulfonium ylide or sulfoxonium ylide produced by intermittently adding sulfonium halide or sulfoxonium halide and a base in the solvent.

Alternatively, in the method for producing a triazolylmethyl cycloalkanol derivative according to the present invention, a base is contained in the solvent, and the sulfur ylide is preferably sulfonium ylide or sulfoxonium ylide produced by intermittently adding sulfonium halide or sulfoxonium halide in the solvent containing the base.

In the method for producing a triazolylmethyl cycloalkanol derivative according to the present invention, the alkali metal salt of 1,2,4-triazole in the reacting step is preferably produced from 1,2,4-triazole and a base containing an alkali metal atom in a reaction system in the reacting step.

Furthermore, in the method for producing a triazolylmethyl cycloalkanol derivative according to the present invention, the reaction temperature is preferably a temperature of higher than 120°C.

Furthermore, in the method for producing a triazolylmethyl cycloalkanol derivative according to the present invention, the reaction temperature is preferably a temperature of 130°C or less.

Furthermore, in the method for producing a triazolylmethyl cycloalkanol derivative according to the present invention, n is preferably 1.

Furthermore, in the method for producing a triazolylmethyl cycloalkanol derivative according to the present invention, the solvent is preferably (i) a polar solvent having an amide bond, (ii) dimethyl sulfoxide, or (iii) a mixed solvent of a polar solvent having an amide bond or dimethyl sulfoxide and alcohol having from 1 to 5 carbons.

Furthermore, in the method for producing a triazolylmethyl cycloalkanol derivative according to the present invention, the polar solvent is preferably N-methyl-2-pyrrolidone, dimethylformamide, or dimethylacetamide.

Furthermore, in the method for producing a triazolylmethyl cycloalkanol derivative according to the present invention, the mixed solvent is preferably a mixed solvent of N-methyl-2-pyrrolidone and tert-butanol.

The present invention is also a triazolylmethyl cycloalkanol derivative-containing composition which is a composition containing a cycloalkanol derivative (II). The triazolylmethyl cycloalkanol derivative-containing composition is obtained by the method for producing a triazolylmethyl cycloalkanol derivative described above.

In the triazolylmethyl cycloalkanol derivative-containing composition according to the present invention, a content of a cycloalkanol derivative (III) is preferably an amount that is 5.0 wt.% or less of the amount of the cycloalkanol derivative (II).

Embodiments of the present invention will be described in further detail hereinafter using working examples. Of course, the present invention is not limited to the working examples below, and it goes without saying that various modes are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents cited in this specification are hereby incorporated by reference.

### EXAMPLES

### (Working Example 1)

In a 500 mL four-neck flask equipped with a Dimroth condenser with a drying tube containing calcium chloride, a stirring equipment with a hermetic seal, a reaction liquid temperature indicator, and a nitrogen introduction tube, 75.1 mL of N-methyl-2-pyrrolidone, 27.15 g of sodium 1,2,4-triazole salt, and 60.81 g of 5-(4-chlorobenzyl)-2,2-dimethylcyclopentanone were added at room temperature in a nitrogen atmosphere, and then 16.00 g of sodium tert-butoxide and 60.75 g of trimethylsulfoxonium bromide were dividedly added respectively at a temperature of higher than 120°C and 130°C or less. After the addition was completed, the mixture was further reacted for 1 hour.

After the obtained reaction liquid was cooled to 80°C, water was added to stop the reaction. Then, the mixture was extracted with toluene. The toluene-extracted liquid was washed, and then the solvent was removed by distillation under reduced pressure to obtain 5-(4-chlorobenzyl)-1-(1H-1,2,4-triazol-1-yl methyl)-2,2-dimethylcyclopentanol as an oily substance. Furthermore, the content of 5-(4-chlorobenzyl)-1-(1H-1,3,4-triazol-1-yl methyl)-2,2-dimethylcyclopentanol (1,3,4-triazole form) was measured by gas chromatography.
The content of the 1,3,4-triazole form: 0.15%

### (Working Example 2)

5-(4-chlorobenzyl)-1-(1H-1,2,4-triazol-1-yl methyl)-2,2-dimethylcyclopentanol was produced in the same manner as in Working Example 1 except for changing the temperature at divided addition to 135°C.
The content of the 1,3,4-triazole form: 0.14%

### (Working Example 3)

5-(4-chlorobenzyl)-1-(1H-1,2,4-triazol-1-yl methyl)-2,2-dimethylcyclopentanol was produced in the same manner as in Working Example 1 except for changing the temperature at divided addition to 115°C.
The content of the 1,3,4-triazole form: 0.52%

### (Working Example 4)

2-(4-chlorobenzyl)-1-(1H-1,2,4-triazol-1-yl methyl)-5-(2-propyl)cyclopentanol was produced in the same manner as in Working Example 1 except for changing the temperature at divided addition to 115°C, and using 64.42 g of 2-(4-chlorobenzyl)-5-(2-propyl)cyclopentanone in place of 5-(4-chlorobenzyl)-2,2-dimethylcyclopentanone.
The content of the 1,3,4-triazole form: 1.24%

### (Comparative Example)

5-(4-chlorobenzyl)-1-(1H-1,2,4-triazol-1-yl methyl)-2,2-dimethylcyclopentanol was produced in the same manner as in Working Example 1 except for changing the temperature at divided addition to 100°C.
The content of the 1,3,4-triazole form: 5.95%

### INDUSTRIAL APPLICABILITY

The present invention can be used for producing triazolylmethyl cycloalkanol derivatives that are active ingredients of agricultural and horticultural fungicides.

## Claims

1. A method for producing a triazolylmethyl cycloalkanol derivative, in which a cycloalkanone derivative represented by general formula (I): 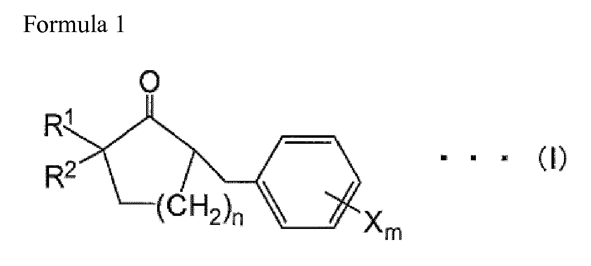 (wherein, R¹ represents a hydrogen atom or an alkyl group or haloalkyl group having from 1 to 5 carbons, R² represents a hydrogen atom or an alkyl group having 1 or 2 carbons, X represents a halogen atom, an alkyl group having from 1 to 4 carbons, a phenyl group, -CN, -CF₃, or -NO₂, m represents an integer of 1 to 5, and n represents an integer of 1 to 3, and if m is 2 or greater, the plurality of X may be different each other)
is azolylmethylated to produce a triazolylmethyl cycloalkanol derivative represented by general formula (II): 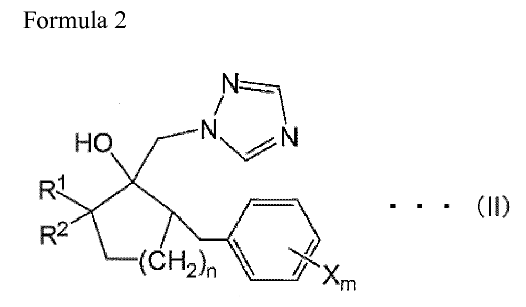 (wherein, R¹, R², X, m, and n are the same as R¹, R², X, m, and n in formula (I) respectively); the method for producing a triazolylmethyl cycloalkanol derivative comprising a step of reacting, in a solvent, the cycloalkanone derivative with an alkali metal salt of 1,2,4-triazole in the presence of sulfur ylide;
a reaction temperature in the reacting step being higher than 110°C and 140°C or lower.

2. The method for producing a triazolylmethyl cycloalkanol derivative according to claim 1, wherein the sulfur ylide in the reacting step is produced in a reaction system of the reacting step.

3. The method for producing a triazolylmethyl cycloalkanol derivative according to claim 2, wherein the sulfur ylide is sulfonium ylide or sulfoxonium ylide produced by intermittently adding sulfonium halide or sulfoxonium halide and a base in the solvent.

4. The method for producing a triazolylmethyl cycloalkanol derivative according to claim 2, wherein the solvent contains a base; and
the sulfur ylide is sulfonium ylide or sulfoxonium ylide produced by intermittently adding sulfonium halide or sulfoxonium halide in the solvent containing the base.

5. The method for producing a triazolylmethyl cycloalkanol derivative according to any one of claims 1 to 4, wherein the alkali metal salt of 1,2,4-triazole in the reacting step is produced from 1,2,4-triazole and a base containing an alkali metal atom in a reaction system in the reacting step.

6. The method for producing a triazolylmethyl cycloalkanol derivative according to any one of claims 1 to 5, wherein the reaction temperature is a temperature of higher than 120°C.

7. The method for producing a triazolylmethyl cycloalkanol derivative according to any one of claims 1 to 6, wherein the reaction temperature is a temperature of 130°C or lower.

8. The method for producing a triazolylmethyl cycloalkanol derivative according to any one of claims 1 to 7, wherein n is 1.

9. The method for producing a triazolylmethyl cycloalkanol derivative according to any one of claims 1 to 8, wherein the solvent is (i) a polar solvent having an amide bond, (ii) dimethyl sulfoxide, or (iii) a mixed solvent of a polar solvent having an amide bond or dimethyl sulfoxide and alcohol having from 1 to 5 carbons.

10. The method for producing a triazolylmethyl cycloalkanol derivative according to claim 9, wherein the polar solvent is N-methyl-2-pyrrolidone, dimethylformamide, or dimethylacetamide.

11. The method for producing a triazolylmethyl cycloalkanol derivative according to claim 9, wherein the mixed solvent is a mixed solvent of N-methyl-2-pyrrolidone and tert-butanol.

12. A triazolylmethyl cycloalkanol derivative-containing composition which is a composition containing a triazolylmethyl cycloalkanol derivative represented by general formula (II): 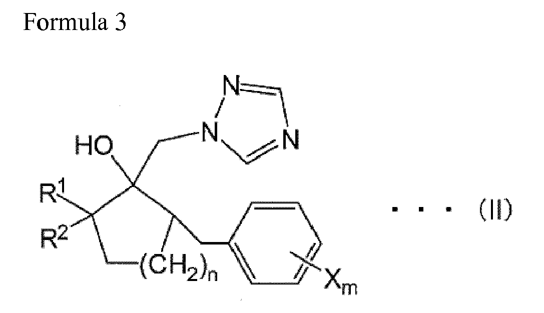 wherein, R¹ represents a hydrogen atom or an alkyl group or haloalkyl group having from 1 to 5 carbons, R² represents a hydrogen atom or an alkyl group having 1 or 2 carbons, X represents a halogen atom, an alkyl group having from 1 to 4 carbons, a phenyl group, -CN, -CF₃, or - NO₂, m represents an integer of 1 to 5, and n represents an integer of 1 to 3, and if m is 2 or greater, the plurality of X may be different each other;
the triazolylmethyl cycloalkanol derivative-containing composition being obtained by the method for producing a triazolylmethyl cycloalkanol derivative described in any one of claims 1 to 11.

13. The triazolylmethyl cycloalkanol derivative-containing composition according to claim 12, wherein a content of a triazolylmethyl cycloalkanol derivative represented by general formula (III): 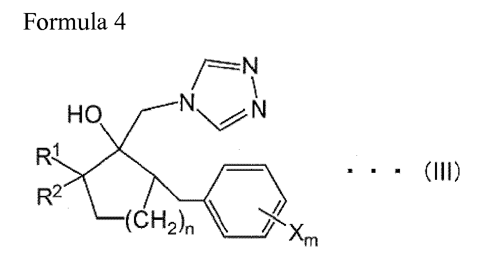 (wherein, R¹, R², X, m, and n are the same as R¹, R², X, m, and n in formula (I) respectively) is an amount that is 5.0 wt.% or less of the amount of the triazolylmethyl cycloalkanol derivative represented by general formula (II) above.
